# EUROPEAN PATENT SPECIFICATION

(11) **EP 0 790 317 B1**
(45) Date of publication and mention of the grant of the patent: **14.05.2003**
(21) Application number: 96914216.5
(22) Date of filing: 24.05.1996
(51) Int. Cl.: C12Q 1/42

(54) **PROCESS BASED ON THE INHIBITION OF PHOSPHATASES FOR DETECTING AND QUANTIFYING DIARRHOETIC SHELLFISH POISON**
AUF DER INHIBIERUNG VON PHOSPHATASEN BERUHENDES VERFAHREN ZUM NACHWEIS UND ZUR QUANTIFIZIERUNG VON DIARRHOETISCHEM SCHALENTIERGIFT
PROCEDE DE DETECTION ET DE QUANTIFICATION DE TOXINES DIARRHEIQUES DES DINOFLAGELLES, AYANT RECOURS A L'INHIBITION DE PHOSPHATASES

(30) Priority: 07.06.1995 ES 9501137
(43) Date of publication of application: 20.08.1997
(73) Proprietor: UNIVERSIDADE DE SANTIAGO DE COMPOSTELA, 15705 Santiago de Compostela (ES)
(72) Inventor: BOTANA LOPEZ, Luis Miguel Universidade de Santiago, Departamento de Farmacoloxia 27002 Lugo (ES); RODRIGUEZ VIEYTES, Mercedes Universidade de, Departamento de Fisioloxia 27002 Lugo (ES); VIEITES BAPTISTA DE SOUSA, Juan Manuel, Campus Universitario E-36310 Vigo (ES); LEIRA SANMARTIN, Francisco ANFACO - CECOPESCA, E-36310 Vigo (ES)
(74) Representative: Davila Baz, Angel
(86) International application number: ES9600117
(87) International publication number: WO96040983

(56) References cited:
- WO-A-92/09891
- J. F. SIMON ET AL.: "Highly sensitive assay of okadaic acid using protein phosphatase and paranitrophenyl phosphate." NATURAL TOXINS, vol. 2, 1994, pages 293-301, XP002010435
- F. A. ANTHONY ET AL.: "A spectrofluorometric assay of calmodulin-dependent protein phosphatase using 4-methylumbelliferyl phosphate." ANALYTICAL BIOCHEMISTRY, vol. 155, 1986, pages 103-107, XP002010436 NEW YORK US
- M. L. BILLINGSLEY.: "Biochemical and molecular characterization of calcineurin (protein phosphatase 2B) in nervous tissue." NEUROPROTOCOLS, US, vol. 6, 1995, pages 20-28, XP002010437
- Q. CHENG ET AL.: "A continuous spectrophotometric assay for protein phosphatases." ANALYTICAL BIOCHEMISTRY, vol. 226, no. 1, 20 March 1995 (1995-03-20), pages 68-73, XP002010438 NEW YORK US

## Description

Diarrheic-producer dinoflagellates show up at certain periods of the year, and their proliferation causes a red tide, which causes the paralyzation of collection and marketing of mollusks in the contaminated zones. The monitoring of the possible mollusk contamination is checked by the mouse bioassay, which consist on the oral administration to mice of a mollusk extract, and their observation for 12 hours. The dead of two out of three innoculed mice in less than 12 hours is considered a positive result of the bioassay.

This method implicates the use of a large amount of mice, which increases the cost of the bioassay, a large investment in man-hours, and it lacks sensitivity. Moreover, it has a large variability, depending on the mouse weight, race, etc. which can be somewhat reduced by using mice of certain features, thus making costs and limitations even higher.
- the document *NATURAL TOXINS, vol.2, 1994, pages 293-301* discloses a colorimetric phosphatase inhibition bioassay for the quantitative measurement of okadaic acid, a toxin responsible for diarrheic shellfish poisoning. The assay uses an artificial substrate, paranitrophenylphosphate, and a semi-purified PP2A prepared from rabbit muscle; this method presents the disadvantage of presenting low sensitivity, possibility of false positives due to interferences of the matrix of the sample with the dye, and needing a complex extraction to avoid interference of colorants from the matrix.
- The document *WO-A-92 09891 (National Council of Canada), 11*^{*th*} *June 1992*, describes a continuous spectrofluorimetric assay of calmodulin-dependent phosphatase, monitoringthe formation of a fluorescent 4-methylumbelliferyl phosphate.

Diarrheic toxins (okadaic acid, dinophysistoxin I, II, III, and the recently identified yessotoxins and pectenotoxins), act by potently and selectively inhibiting cytosolic phosphatases PP1 and PP2A. These phosphatases have been characterized several years ago, and their obtention in high amounts is possible, by using sources rich in phosphatases, such as skeletetic muscle or brain. The in vitro study of the inhibitory effect of DSP toxins on phosphatases has been therefore already described by means of the use of radioactive markers, which in no way could be used for a detection method due to the problems that phosphorous radiation has, as well as its short half life, cost, and high degree of error associated to this procedure.

Phosphatases can be purified from a tissue homogenate by ionic exchange chromatography (eluting the mobile phase with low pressure), and subsequent repurification of the obtained fractions which contain the phosphatases.

This procedure can be continued until total purification of each enzyme, although it is very tedious and a delicate process. The phosphatases obtained are active and can be preserved at low temperatures for up to 6 months. This purification procedure can be speeded and improved by using higher pressure systems, thus reducing the separation time and increasing the efficacy of the separation.

Phosphatases can be used without the need to reach a total purification, so that they maintain the sensitivity to the inhibitory effect of the DSP toxins but with a lower effort and cost of purification. The enriched phosphatases are placed in a microtiter plate, and the inhibition assay to determine the presence or not of the toxins is carried out by incubating in the plate wells a 80 % methanolic extract in the presence of a certain amount of enzyme. The toxins are determined by comparing the hosphatase activity with known standards of okadaic acid (which is the main diarrheic toxin). The quantification of the toxin activity is by quantification of the color of free phosphate, the fluorescence (in the case of fluorescent substrates), or the luminescence (in the case of luminescent substrates) in a spectrophotometer, fluorimeter or lumminometer, respectively, which generate the different types of phosphatase PP2A substrates.

The advantages of this technique are:
- It does not require the use of mice.
- It does not require an observation time.
- It corrects the lack of sensitivity and specificity of the bioassay.
- The preparation time of the assay is 30 minutes.
- The time to obtain the results is 5 minutes.
- Several hundred samples can be processed simultaneously.
- The cost is lower than the cost of current techniques.
- It can be performed by personnel not highly qualified.
- It lacks the inconvenients of other techniques based on the use of radioactive markers or on the chromatographic detection of the toxins (high cost, lack of repetitivity, risk of radioactivity, residues).

A way to carry out the invention:

Obtention by chromatographic techniques of PP2A phosphatase-enriched fractions:

A rabbit is sacrificed with an anesthetic overdose. The muscles are isolated from the back and the legs, and they are kept on cold. The tissue is homogenized after being cut, in a buffer at pH 7,4. The homogenate is centrifuged at 100.000 g for 1 hr, and the supernatants are collected.

The supernatant is loaded onto a DEAE-cellulose column, which was previously activated, and it is eluted with a sodium chloride gradient from 0 to 0.4 molar, buffered with 500 mM Tris HCL at pH 7.4 and with 10 %-glycerol.

The obtained peaks are detected in a spectrophotometer at 600 nm. Six major absorbance peaks are obtained, of which the second and third are enriched with PP2A. The phosphatase activity is checked by measuring the color-generated by the phosphate released from the serine/threonine phosphopeptide. The activity is checked against a calibration curve obtained with free phosphate.

Determination of the presence of DSP toxins in contaminated samples.

The development of this technique was carried out by using, independently and with similar results, the following PP2A-phosphatase sources:
1) The catalytic subunit of protein phosphatase type 2A (PP2A) from rabbit muscle, and with a purity higher than 70 %, obtained from a commercial source.
2) The protein phosphatase type 2A (PP2A) from human red cells isolated as the heterodimer with the subunits A (60kDa) and B (36kDa), obtained from a commercial source.
3) The chromatographic fractions enriched in PP2A and obtained by partial purification from rabbit muscle.

The assay is carried out in a plate of microtitration with 96 wells, where PP2A is incubated with the reaction buffer (50 mM Tris HCL, pH 8.5) at 37 degrees Celsius, for 15 minutes in a volume of 50-80 µL. After the 15 min an aliquot is added to different dilutions of the methanolic extract obtained from DSP-contaminated mollusk (the obtained extract is dried up, resuspended in 10 % DMSO (dimethylsulphoxide) before its use) and incubated again for a few minutes at 37 degrees Celsius. In order to measure the PP2A activity, the enzyme substrate is added in a volume of 150-180 µL in 50 mM Tris HCL, pH 8.5, to obtain a final volume of 200 µL. In the case of a fluorimetric technique, in order to measure the activity, a fluorescent substrate of the enzyme is added, such as 70 µM methylumbeliferone phosphate, or 30 µM fluorescein diphosphate. If colorimetric technique is used, a specific substrate of the enzyme is added, such as the phosphopeptide serine/threonine, and then the release of free phosphate is quantified in a spectrophotometer at a wavelength of 600 nm.

The activity of phosphatase PP2A will be inhibited proportionally to the amount of DSP toxins. In order to quantify the toxins, it is required a calibration curve obtained with known amounts of okadaic acid, where the fluorescence or optical density data obtained from the wells with the samples gives the concentration of DSP toxins able to inhibit the phosphatase activity. Depending on the substrate, which can be colored, fluorescent or luminescent due to the release of phosphate groups, there will be three types of non radioactive techniques, namely espectrophotometric, luminometric or fluorimetric, to detect DSP toxins.

In order to carry out these non radioactive techniques, it is necessary to use compounds with two main features:
1) They are good substrates of PP2A phosphatase.
2) The solvents used to obtain the extract (methanol) do not interfere with the determination.

Good substrates having these two features, critical to perform a routine DSP toxin assay are the following:
1) Phosphate of 4-Methylumbelliferona (4-MUP). Excitation 370/Emission 430
2) Diphosphate of fluorescein (FDP). Excitation 480/Emission 520.
3) A serine/threonine phosphopeptide. The reading of the optical density must be done in microtiter plates at a wavelength of 600 nm.

## Claims

1. Procedure for the detection and quantification of diarrheic toxins of dinoflagellates (DSP (diarrheic shellfish poison)) based upon the inhibition of phosphatases, by measuring the activity of PP2A phosphatase, obtained from human red cells or after their partial purification from a tissue that is rich in these enzymes, with fluorescent PP2A phosphatase substrates which are sensitive to the action of the phosphatase and that undergo a quantifiable change in their fluorescence.

2. Procedure according to claim 1, **characterized in that** the fluorescent PP2A phosphatase substrates are selected from the group consisting of 4-Methytumbelliferona (4-MUP) and diphosphate of fluorescein (FDP).

## Patentansprüche

1. Verfahren zur Ermittlung und Quantifizierung diarrhöischer Toxine von Dinoflagellaten (DSP (diarrhöisches Schalentiergift)) basiert auf Inhibition von Phosphatasen, durch Messung der Aktivität von PP2A Phosphatase, die aus menschlichen roten Blutkörperchen oder nach ihrer teilweisen Reinigung von einem an diesen Enzymen reichen Gewebe mit fluoreszierenden PP2A Phosphatasesubstraten gewonnen werden, mit fluoreszierenden PP2A Phosphatasesubstraten, die empfindlich gegenüber der Wirkung von Phosphatase sind und die eine quantifizierbare Veränderung ihrer Fluoreszenz erleben.

2. Verfahren gemäss Patentanspruch 1, **gekennzeichnet dadurch, dass** die fluoreszierenden PP2A Phosphatasesubstrate aus der Gruppe bestehend aus Methylumbelliferon (4-MUP) und Fluoresceindiphosphat (FDP) ausgewählt werden.

## Revendications

1. Procédure pour le dépistage et la quantification de toxines diarrhéiques des dinoflagellés (DSP - intoxication diarrhéique due aux coquillages) basée sur l'inhibition des phosphatases en mesurant l'activité de la phosphatase PP2A, à partir de cellules rouges humaines ou après leur dépuration partielle d'après un tissu riche en ces enzymes, avec des substrats de phosphatase PP2A fluorescents sensibles à l'action de la phosphatase et qui subissent une modification quantifiable sur leur fluorescence.

2. Procédure selon la revendication 1, **caractérisée par le fait que** les substrats de phosphatase PP2A fluorescents se sélectionnent du groupe consistant de 4-méthylumbelliferone (4-MUP) et du diphosphate de fluoresceine (FDP).
